# EUROPÄISCHE PATENTSCHRIFT

(11) **EP 4 427 698 B1**
(45) Veröffentlichungstag und Bekanntmachung des Hinweises auf die Patenterteilung: **07.05.2025**
(21) Anmeldenummer: 23211895.0
(22) Anmeldetag: 24.11.2023
(51) Int. Cl.: A61B 50/00, A61B 50/31, A45C 13/02, A45C 13/08

(54) **RUCKSACK ODER KOFFER**
RUCKSACK OR SUITCASE
SAC À DOS OU VALISE

(30) Priorität: 06.03.2023 DE 202023101062 U
(43) Veröffentlichungstag der Anmeldung: 11.09.2024
(73) Patentinhaber: hestomed GmbH, 09356 St. Egidien (DE)
(72) Erfinder: Helms, Steffen, 09244 Lichtenau (DE); Rothschild, Dirk, 09127 Chemnitz (DE)
(74) Vertreter: Steiniger, Carmen

(56) Entgegenhaltungen:
- DE-B3- 102014 106 735
- DE-U1- 202015 101 973
- DE-U1- 9 012 093
- US-A- 3 306 405

## Beschreibung

Die vorliegende Erfindung betrifft einen Rucksack oder Koffer mit einer Unterschale und einer klappbar mit der Unterschale verbundenen Oberschale, wobei sowohl die Unterschale als auch die Oberschale jeweils eine Innenlage und eine Außenlage aufweisen.

Rucksäcke oder Koffer, insbesondere für die Aufbewahrung von medizinischer Ausrüstung, wie z. B. Notfalltaschen oder -rucksäcke für den Rettungsdienst, weisen typischerweise spezifische Aufnahmen für medizinische Ausrüstung auf. Dazu zählen beispielsweise innenseitig des Rucksackes oder Koffers vorgesehene Halterungen für medizinische Apparate sowie Fixierflächen für herausnehmbare Innentaschen oder Instrumente, die im Inneren des Rucksackes oder Koffers an entsprechenden Positionen über geeignete Verbindungsmittel lösbar fixiert sind.

Zweckmäßigerweise ist die Anordnung der Aufnahmen im Inneren des Rucksackes oder Koffers variabel, um ein optimales und übersichtliches Bepacken des Rucksackes oder des Koffers zu ermöglichen.

Die Fixierung von Innentaschen sowie medizinischer Apparate und Instrumente im Inneren eines Rucksackes oder Koffers erfolgt häufig über Klettverschlüsse. Über diese können Ausrüstungsgegenstände positionsfest im Inneren des jeweiligen Rucksackes oder Koffers angeordnet werden. Klettverschlüsse nutzen sich jedoch bei häufigem Gebrauch relativ schnell ab und sind anfällig für Verschmutzung. Eine gründliche Reinigung und Desinfektion der Klettverschlüsse ist sehr aufwendig.

Aus dem Stand der Technik sind zudem Transportbehälter mit magnetischen Fixierflächen bekannt. So offenbart die Druckschrift DE 20 2014 000 119 U1 beispielsweise einen Rettungsrucksack, in dem sich Ausstattungselemente mittels Magneten flexibel anordnen und fixieren lassen.

Aus der Druckschrift DE 20 2015 101 973 U1 ist ein Transportbehälter für medizinische Ausrüstung bekannt, wobei der Transportbehälter in einer Unterschale des Transportbehälters angeordnete Fixierflächen aufweist. Die Fixierflächen weisen jeweils einen Hohlraum auf, in dem jeweils ein Magnetbefestigungselement angeordnet ist. Das jeweilige Magnetbefestigungselement ist in dem jeweiligen Hohlraum in einer Ebene parallel zu der Fixierfläche frei verschiebbar. Das heißt, das Magnetbefestigungselement liegt schwimmend in dem Hohlraum, der deutlich größer ausgebildet ist, als es für eine Aufnahme des Magnetbefestigungselementes erforderlich ist. Die Hohlräume sind durch ein Vernähen oder Verschweißen zweier aufeinander liegender Materialschichten gebildet. An den Fixierflächen lässt sich eine herausnehmbare Einlegetasche lösbar über eine Magnetverbindung fixieren.

Durch die schwimmend in den Hohlräumen angeordneten Magnetbefestigungsmaterialien lassen sich Ausrüstungsgegenstände, die in dem Transportbehälter aufgenommen werden sollen, nicht ortsfest fixieren, wodurch die Ausrüstungsgegenstände verrutschen und durcheinander geraten können. Zudem bilden die miteinander verschweißten oder vernähten Schichten, zwischen den die Magnetbefestigungselemente angeordnet sind, eine schmutzanfällige und schwer zu reinigende Innenlage des Transportbehälters.

Auch die Druckschrift DE 20 2018 101 350 U1 beschreibt einen Rettungskoffer, welcher zur Fixierung von Modultaschen in dem Rettungskoffer einen Boden mit Magnetelementen aufweist. Zum Befestigen der Modultaschen an dem Boden des Rettungskoffers sind an einer Unterseite der Modultaschen ebenfalls Magnetelemente angebracht.

Davon ausgehend ist es die Aufgabe der vorliegenden Erfindung, einen Rucksack oder Koffer zur Verfügung zu stellen, der leichter zu reinigen und zu desinfizieren ist und der eine sichere und präzisere Anordnung von Ausrüstungsgegenständen innerhalb des Rucksackes oder Koffers ermöglicht.

Erfindungsgemäß wird diese Aufgabe durch einen Rucksack oder Koffer gemäß Anspruch 1 gelöst.

Der erfindungsgemäße Rucksack oder Koffer weist eine Unterschale und eine Oberschale auf, wie sie beispielsweise von einem Hartschalenkoffer oder einem Hartschalenrucksack bekannt sind. Die Oberschale und die Unterschale sind klappbar miteinander verbunden, sodass sich der Rucksack oder Koffer auf- und zuklappen lässt.

Sowohl die Unterschale als auch die Oberschale weisen jeweils eine Innenlage und eine Außenlage auf, wobei die Innenlage auf einer einem Inneren des Rucksackes oder Koffers zugewandten Seite der Unterschale und auf einer einem Inneren des Rucksackes oder Koffers zugewandten Seite der Oberschale angeordnet ist. Entsprechend ist die Außenlage auf einer dem Inneren des Rucksackes oder Koffers abgewandten Seite der Unterschale und auf einer dem Inneren des Rucksackes oder Koffers abgewandten Seite der Oberschale angeordnet. Die Innenlage und die Außenlage können aus demselben Material und/oder Materialverbund oder aus unterschiedlichen Materialien und/oder Materialverbünden ausgebildet sein.

Beispielsweise sind die Außenlage und die Innenlage als Kunststoffformteile oder Kunststoffspritzteile oder aus jeweils einer thermoplastischen Polyurethan-Plane (TPU-Plane) ausgebildet. Vorzugsweise ist die Polyurethan-Plane aus einem Nylongewebe mit einer darauf aufgebrachten thermoplastischen Polyurethanbeschichtung ausgebildet. Es ist jedoch auch möglich, dass nur die Innenlage als Kunststoffformteil oder Kunststoffspritzteil oder einer Polyurethan-Plane ausgebildet ist. Wenn zwischen der Innenlage und der Außenlage eine Kunststoffplatte angeordnet ist, sind vorzugsweise die Außenlage und die Innenlage jeweils aus einer thermoplastischen Polyurethan-Plane ausgebildet.

Die Oberschale und/oder die Unterschale weist/weisen magnetische Kopplungselemente auf, an denen sich Ausrüstungsgegenstände, die in dem Rucksack oder Koffer aufgenommen werden sollen und die selbst magnetische Kopplungselemente aufweisen, lösbar fixieren lassen.

Bei den Ausrüstungsgegenständen handelt es sich vorzugsweise um medizinische Apparate und Instrumente sowie Einlegetaschen und/oder -module, die wiederum unterschiedlich bestückt sein können.

Die magnetischen Kopplungselemente sind entweder Magnete oder magnetische Materialien. Beispielweise sind die magnetischen Kopplungselemente Permanentmagnete und die Ausrüstungsgegenstände, die in dem Rucksack oder Koffer aufgenommen werden sollen, weisen ferromagnetische Elemente auf, die mit den Permanentmagneten zusammenwirken, um eine lösbare Magnetverbindung auszubilden. Gleichfalls ließen sich beispielsweise die magnetischen Kopplungselemente durch ferromagnetische Materialien ausbilden und die Ausrüstungsgegenstände entsprechend mit Permanentmagneten ausstatten. In einer weiteren Ausführungsform des erfindungsgemäßen Rucksackes oder Koffers können sowohl der Rucksack oder Koffer als auch die Ausrüstungsgegenstände Permanentmagnete aufweisen.

In einem Schnitt durch die Oberschale und/oder Unterschale weisen die magnetischen Kopplungselemente eine vorzugsweise nicht bedeckte Oberfläche auf, die einer Innenseite der Außenlage gegenüberliegt.

Vorzugsweise sind die magnetischen Kopplungselemente als Stahlscheiben ausgebildet. Die magnetischen Kopplungselemente können jedoch auch Magnetscheiben sein.

Erfindungsgemäß weist die Innenlage der Oberschale und/oder der Unterschale eine Kunststoffwanne mit Vertiefungen auf, in welchen die magnetischen Kopplungselemente bündig aufgenommen sind. Dabei sind die Vertiefungen derart ausgebildet, dass sich die jeweils darin befindlichen magnetischen Kopplungselemente zwischen der Außenlage und der Innenlage der Oberschale und/oder zwischen der Außenlage und der Innenlage der Unterschale befinden. Das heißt, die magnetischen Kopplungselemente sind zwischen der Innenlage und der Außenlage verborgen angeordnet. Hierzu sind die Vertiefungen in einer zu der Außenlage ausgerichteten Seite der Innenlage eingebracht.

Die Vertiefungen erstrecken sich ausgehend von der zur Außenlage ausgerichteten Seite der Innenlage in Richtung eines Inneren des Rucksackes oder Koffers.

Zudem sind die magnetischen Kopplungselemente, dadurch dass sie bündig in den Vertiefungen aufgenommen sind, lagefixiert angeordnet und können nicht verrutschen. Hierbei ist unter bündig zu verstehen, dass die magnetischen Kopplungselemente und die Vertiefungen derart ausgebildet sind, dass die magnetischen Kopplungselemente jeweils beidseitig an zu den magnetischen Kopplungselementen ausgerichteten Seitenrändern der Vertiefungen angrenzen. Die magnetischen Kopplungselemente sind passgenau zu den Vertiefungen ausgebildet. Die passgenaue Ausbildung der magnetischen Kopplungselemente verhindert ein Verrutschen der magnetischen Kopplungselemente in den Vertiefungen. Ferner ragen die bündig in den Vertiefungen aufgenommenen magnetischen Kopplungselemente kaum oder gar nicht aus den Vertiefungen in der Kunststoffwanne heraus, wodurch sie sich nicht in der angrenzenden oder gegenüber befindlichen Außenlage abzeichnen oder die Außenlage nach außen aufwölben.

Die magnetischen Kopplungselemente sind in den Vertiefungen der Kunststoffwanne aufgenommen und zwischen der Innenlage und der Außenlage der Oberschale und/oder Unterschale verborgen angeordnet.

Die Vertiefungen ragen vorzugsweise in das Innere des Rucksackes oder Koffers. Das heißt, auf einer zu dem Inneren des Rucksackes oder Koffers gerichteten Seite der Innenlage bilden sich Noppen ab, hinter denen sich jeweils die magnetischen Kopplungselemente befinden. Dadurch sind die magnetischen Kopplungselemente an der zu dem Inneren des Rucksackes oder Koffers gerichteten Innenseite der Innenlage bedingt durch die Vertiefungen in ihren Umrissen lokalisierbar. Damit wird eine zuverlässige Positionierung und Fixierung von Ausrüstungsgegenständen in dem erfindungsgemäßen Rucksack oder Koffer ermöglicht.

Die Kunststoffwanne ist leicht und widerstandsfähig zugleich. Zudem verleiht sie der Oberschale und/oder Unterschale eine stabile Form, wodurch sich auch empfindliche Ausrüstungsgegenstände sicher in der jeweiligen Oberschale und/oder Unterschale aufnehmen und vor Beschädigungen schützen lassen.

Vorzugsweise handelt es sich bei der Kunststoffwanne um ein einteiliges Tiefzieh- oder Spritzgussteil.

Im Vergleich zu einer textilen Innenlage, die zerknittern und verrutschen kann, sorgt die Kunststoffwanne für eine glatte, stabile, fugen- und nahtlose Innenlage der Ober- und/oder Unterschale, die unempfindlich gegenüber Schmutz ist und die sich leicht reinigen und desinfizieren lässt.

Zwischen den magnetischen Kopplungselementen und der Außenlage kann sich in einfachen Ausführungsformen der Erfindung Luft befinden. In einer speziellen Ausführungsform des erfindungsgemäßen Rucksacks oder Koffers ist zwischen der Außenlage und der Kunststoffplatte wenigstens eine Schaumplatte angeordnet. Durch die wenigstens eine Schaumplatte werden in dem erfindungsgemäßen Rucksack oder Koffer befindliche Ausrüstungsgegenstände gut gegen Stöße geschützt.

Je nachdem wie die magnetischen Kopplungselemente in der Oberschale und/oder der Unterschale angeordnet sind, lassen sich die jeweilige Oberschale und/oder Unterschale mit magnetischen Elementen aufweisenden Ausrüstungsgegenständen bestücken, wobei diese über Magnetverbindungen an der Oberschale und/oder Unterschale fixiert sind.

Der erfindungsgemäße Rucksack oder Koffer eignet sich insbesondere zum Transport medizinischer Ausrüstungsgegenstände, wie sie beispielsweise im Rettungsdienst und in der Notfallmedizin benötigt werden.

In einer bevorzugten Ausführungsform der Erfindung sind die magnetischen Kopplungselemente in die Vertiefungen geklebt. Dadurch wird verhindert, dass die magnetischen Kopplungselemente aus den Vertiefungen herausrutschen können und diese auch bei einer starken Materialbeanspruchung des Rucksackes oder Koffers bei rauen Einsatzbedingungen in ihrer Lage fixiert bleiben. Insofern die magnetischen Kopplungselemente nicht in die Vertiefungen eingeklebt sind, werden diese vorzugsweise durch eine formschlüssige Verbindung in den Vertiefungen gehalten. Hierbei sind die magnetischen Kopplungselemente passgenau zu den Vertiefungen ausgebildet. In einer weiteren Ausführungsform der Erfindungen können die magnetischen Kopplungselemente auch durch das Anliegen der magnetischen Kopplungselemente an die Außenlage in den in der Innenlage eingebrachten Vertiefungen gehalten werden. Auch bei einer solchen Ausführungsform ist die Form der Kopplungselemente an die Form der Vertiefungen angepasst.

Vorzugsweise ist die Außenlage der Oberschale und/oder der Unterschale aus einem Kunststoffformteil oder einem kunststoffbeschichteten Planenmaterial ausgebildet. Eine aus einem Kunststoffformteil oder einem kunststoffbeschichteten Planenmaterial ausgebildete Außenlage ist besonders strapazierfähig und ebenfalls leicht zu reinigen und zu desinfizieren. Eine derartig ausgebildete Außenlage bietet zudem einen hohen Schutz vor Spritzwasser. Eine aus einem Kunststoffformteil ausgebildete Außenlage der Oberschale und/oder der Unterschale verleiht dem Rucksack oder Koffer zudem zusätzliche Stabilität und schützt in dem Rucksack oder Koffer aufgenommene Ausrüstungsgegenstände besonders gut vor Beschädigungen.

Es erweist sich als sehr vorteilhaft, wenn die Innenlage und die Außenlage der Oberschale und der Unterschale flüssigkeitsdicht miteinander verklebt sind. Ebenso können die Innenlage und die Außenlage der Oberschale und/oder Unterschale auch durch Verschweißen flüssigkeitsdicht miteinander verbunden sein. Durch ein Verkleben oder Verschweißen der Außenlage und der Innenlage können sich diese nicht voneinander lösen oder gegeneinander verrutschen. Eine flüssigkeitsdichte Verklebung oder Verschweißung der Außenlage und der Innenlage verhindert auch, dass Schmutz und Feuchtigkeit zwischen die Außenlage und die Innenlage gelangen kann. Damit lässt sich der erfindungsgemäße Rucksack oder Koffer desinfizieren, ohne dabei beschädigt zu werden.

Der Rucksack oder Koffer lässt sich besonders gut verschließen, wenn ein Rand der Unterschale und ein Rand der Oberschale zusammen eine Nut-Feder-Verbindung ausbilden. Die Nut-Feder-Verbindung schließt den Rucksack oder Koffer schmutz- und feuchtigkeitsdicht ab und ist dabei hygienischer und leichter zu reinigen als ein gleichwirkender Klettverschluss oder Reißverschluss.

Für einen einfachen Transport und eine einfache Handhabung des Rucksackes oder Koffers erweist es sich als besonders vorteilhaft, wenn der Rucksack oder Koffer wenigstens einen Tragegriff aufweist, der mit wenigstens einer zwischen der Innenlage und der Außenlage der Oberschale und/oder der Unterschale angeordneten Verstärkungsplatte verschraubt ist. Die Verstärkungsplatte ist zwischen der Innenlage und der Außenlage der Oberschale und/oder der Unterschale verborgen angeordneten, sodass eine ebenmäßige und leicht zu reinigende Oberfläche der Innenlage und der Außenlage durch die Verstärkungsplatte nicht durch Nähte oder Fugen beeinträchtigt wird. In die Verstärkungsplatte lassen sich Schrauben direkt, das heißt, ohne zusätzliche Muttern, eindrehen. Dazu schneiden sich die Schrauben beispielsweise direkt in ein Material der Verstärkungsplatte oder die Verstärkungsplatte weist entsprechend ausgebildete Bohrungen mit einem Innengewinde auf.

Vorzugsweise weist der erfindungsgemäße Rucksack oder Koffer einen Tragegriff auf, der aus nur einem Materialstreifen ausgebildet ist und mittels einer Rastnase in einer zwischen der Innenlage und der Außenlage der Oberschale und/oder der Unterschale angeordneten Tragegriff-Anbindungsplatte angeordnet ist.

Vorzugsweise weist der Rucksack oder Koffer wenigstens eine Reflexionsfläche auf, um auch nachts im Scheinwerferlicht besonders auffällig zu sein.

In einer bevorzugten Ausführungsform der Erfindung weist der Rucksack oder Koffer wenigstens einen Schultergurt auf, der aus nur einem Materialstreifen ausgebildet ist. Das heißt, der Schultergurt ist einstückig ausgebildet und er weist keine Nähte oder Klebefugen auf. Dadurch ist er schmutzunempfindlich und lässt sich leicht reinigen und desinfizieren.

Wenn der Rucksack oder Koffer wenigstens einen Schultergurt aufweist, lässt sich der Tragekomfort des Rucksackes oder Koffers dadurch erhöhen, dass der wenigstens eine Schultergurt anatomisch vorgeformt ist. Das heißt, der eine Materialstreifen, aus dem der wenigstens eine Schultergurt ausgebildet ist, ist derart gebogen, dass er bereits in einem unbeanspruchten Zustand eine an eine Anatomie eines Trägers des Rucksackes oder Koffers abgepasste Form aufweist.

In einer vorteilhaften Ausführungsform des erfindungsgemäßen Rucksackes oder Koffers ist der wenigstens eine Schultergurt mittels einer Schultergurtbefestigungsplatte an der Unterschale des Rucksackes oder Koffers befestigt.

Die Schultergurtbefestigungsplatte kann auf eine Unterschalenaußenseite des Rucksackes oder Koffers aufgebracht sein.

In einer vorteilhaften Ausführungsform des erfindungsgemäßen Rucksackes oder Koffers ist die auf die Unterschalenaußenseite aufgebrachte Schultergurtbefestigungsplatte von einer TPU-Plane überdeckt. Die TPU-Plane kann nur im Bereich der Schultergurtbefestigungsplatte auf die Unterschalenaußenseite aufgebracht sein oder sich über die gesamte Unterschalenaußenseite erstrecken. Die Unterschale ist vorzugsweise einschließlich der Schultergurtbefestigungsplatte von der TPU-Plane überspannt.

In anderen Ausführungsformen der Erfindung kann die Schultergurtbefestigungsplatte jedoch auch zwischen der Innenlage und der Außenlage der Unterschale oder auf einer Innenseite der Unterschale angeordnet sein.

Die Schultergurtbefestigungsplatte ist vorzugsweise auf die Unterschale geklebt, kann aber auch mit der Unterschale verschraubt sein. Durch das Verkleben der Schultergurtbefestigungsplatte mit der Unterschale wird die Unterschale nicht beschädigt, wodurch der erfindungsgemäße Rucksack oder Koffer besonders stabil ausgebildet ist. Wird die Schultergurtbefestigungsplatte mit dem daran befestigten wenigstens einen Schultergurt zwischen der Außenlage und der Innenlage der Unterschale angeordnet, ergibt sich eine besonders feste und langlebige Verbindung des wenigstens einen Schultergurtes mit der Unterschale.

Die Schultergurtbefestigungsplatte ist vorzugsweise aus Aluminium ausgebildet, kann aber auch aus anderen Materialien, wie beispielsweise Kunststoff, Edelstahl oder Titan, ausgebildet sein.

In einer vorteilhaften Ausführungsform des erfindungsgemäßen Rucksackes oder Koffers ist im Bereich der Schultergurtbefestigungsplatte, auf der die Schultergurtbefestigungsplatte überdeckenden TPU-Plane eine Dämpfungsplatte angeordnet. Die Dämpfungsplatte und die Schultergurtbefestigungsplatte können beispielsweise als ZweiKomponenten-Bauteil ausgebildet sein. In Ausführungsform, bei denen die Schultergurtbefestigungsplatte von einer TPU-Plane und einer Dämpfungsplatte überdeckt werden, ist der wenigstens eine Schultergurt vorzugsweise durch Schrauben, die durch die Dämpfungsplatte und die TPU-Plane verlaufen, an der Schultergurtbefestigungsplatte befestigt.

In einer anderen Ausführungsform des erfindungsgemäßen Rucksackes oder Koffers ist die Schultergurtbefestigungsplatte in eine auf die Unterschalenaußenseite der Unterschale ausgebildete Absenkung geklebt. Die Schultergurtbefestigungsplatte ist also dauerhaft mit der Unterschalenaußenseite der Unterschale verklebt. Bei dieser Befestigungsart wird die Unterschale nicht geschwächt oder beschädigt, insbesondere bleibt auch die Innenlage der Unterschale unversehrt. Gleichzeitig garantiert eine vollflächige Verklebung der Schultergurtbefestigungsplatte mit der Unterschale eine widerstandsfähige und zuverlässige Verbindung. Dadurch dass die Schultergurtbefestigungsplatte in eine auf der Unterschalenaußenseite der Unterschale ausgebildete Absenkung geklebt ist, steht sie nicht vor, sodass ein Tragekomfort des Rucksackes oder Koffers nicht beeinträchtigt wird und sich kein Schmutz an überstehenden Kanten ablagern kann.

Wenn der Rucksack oder Koffer eine in die auf der Unterschalenaußenseite der Unterschale ausgebildete Absenkung eingebrachte Schultergurtbefestigungsplatte aufweist, ist es für einen besonders hohen Tragekomfort äußerst vorteilhaft, wenn die Schultergurtbefestigungsplatte als ein Zweikomponenten-Spritzguss-Teil mit einer Halteplatte und einer flächig damit verbundenen Dämpfungsplatte ausgebildet ist, wobei die Halteplatte an der Unterschalenaußenseite angeordnet ist.

Wenn der Rucksack oder Koffer einen Schultergurt aufweist, erweist es sich als sehr geschickt, wenn an dem wenigstens einen Schultergurt eine einstückig mit dem Schultergurt ausgebildete Kederschiene ausgebildet ist. An der Kederschiene lassen sich optionale Ausrüstungsgegenstände, wie beispielsweise eine Taschenlampe oder eine Trinkflasche, aufnehmen und entlang des Schultergurtes in eine für den Träger komfortable Position verschieben. Dadurch dass die Kederschiene einstückig mit dem Schultergurt ausgebildet ist und nicht mit diesem verklebt oder vernäht ist, ist die Kederschiene nahtlos mit dem Schultergurt verbunden. Dies erleichtert eine Reinigung des Rucksackes oder Koffers. Eine einstückige Ausbildung der Kederschiene zusammen mit dem Schultergurt erfolgt vorzugsweise durch ein Gussverfahren.

Wenn der Rucksack oder Koffer wenigstens einen Schultergurt aufweist, an dem eine einstückig mit dem Schultergurt ausgebildete Kederschiene ausgebildet ist, ist es besonders vorteilhaft, wenn ein Brustgurt an der Kederschiene befestigt ist, an dem ein Magnetverschluss ausgebildet ist, wobei der Brustgurt aus einem dehnbaren Material ausgebildet ist und eine Längenverstellung des Brustgurtes ausschließlich über die Dehnbarkeit dieses Materials realisiert wird. Der Brustgurt lässt sich leicht entlang der Kederschiene in eine für einen Träger angenehme Position verschieben. Durch den Magnetverschluss lässt sich der Brustgurt besonders einfach öffnen und schließen. Zudem ist der Magnetverschluss unanfällig gegenüber Verschmutzung. Dadurch dass eine Längenverstellung des Brustgurtes ausschließlich über die Dehnbarkeit des Materials realisiert wird, schmiegt sich der Brustgurt eng an eine Brust des Trägers an und sorgt so für einen sicheren Sitz des Rucksackes oder Koffers. Durch die Dehnbarkeit des Materials wird der Träger in seiner Bewegungsfreiheit sowie beim Atmen kaum beeinträchtigt. Durch die Dehnbarkeit des Materials passt sich der Brustgurt zudem automatisch an eine Statur des Trägers an, sodass er nicht verstellt werden muss.

Es erweist sich als besonders vorteilhaft, wenn die Dämpfungsplatte der Schultergurtbefestigungsplatte, der Schultergurt, der wenigstens eine Tragegriff, der Brustgurt und Zugfähnchen, soweit vorhanden, jeweils in einem Stück aus einem geschlossenporigen, flexiblen und dehnbaren Material ausgebildet sind. Die Ausbildung der genannten Elemente aus einem geschlossenporigen, flexiblen und dehnbaren Material sorgt dafür, dass sich diese an eine Anatomie eines Trägers anpassen, ohne dass sie dazu erst verstellt werden müssten. Dies erleichtert zum einem eine Handhabung des Rucksackes oder Koffers, zum andern entbehrt diese Art der Ausbildung mehrteilige und schwer zu reinigende Verstellmechanismen. Ein Materialstrang oder -stück aus einem geschlossenporigen Material weist zudem einen besonders hohen Tragekomfort auf und ist gleichzeitig einfach zu reinigen und zu desinfizieren.

In einer Ausführungsform des erfindungsgemäßen Rucksackes oder Koffers ist der Rucksack oder Koffer mittels eines an der Ober- und Unterschale angebrachten Reißverschlusses verschließbar.

In einer weiteren Ausführungsform des erfindungsgemäßen Rucksackes oder Koffers sind an aneinandergrenzenden Seiten der Unterschale und der Oberschale miteinander korrespondierende Teile eines Magnetschnappverschlusses montiert. Der Magnetschnappverschluss lässt sich besonders einfach öffnen und schließen, was eine routinierte und sichere Handhabung des Rucksackes oder Koffers auch in Stresssituationen begünstigt.

Die Handhabung des Rucksackes oder Koffers lässt sich in einer vorteilhaften Ausführungsform dadurch erhöhen, dass an einem Rand der Oberschale und/oder einem Rand der Unterschale eine Zugschlaufe montiert ist.

In einer besonders bevorzugten Ausführungsform der Erfindung ist/sind zwischen der Außenlage und der Innenlage der Oberschale und/oder der Unterschale ein RFID- und/oder ein NFC-Transponder angeordnet. Der RFID- und/oder der NFC-Transponder lässt/lassen sich beispielsweise digital mit einer Seriennummer beschreiben. Dies ermöglicht eine eindeutige Identifikation jedes einzelnen Rucksackes oder Koffers. Zudem lassen sich Informationen, wie beispielsweise Produktionsdatum, Produktionsmitarbeiter, Ausstattung, Farbe, Typ, Nutzer, Besteller, etc. eines jeden Rucksackes oder Koffers auslesen und mit einer Datenbank verknüpfen. Ebenso lassen sich beispielsweise Reinigungshinweise, Pflegeanleitungen, Packlisten, Wartungs- und Prüfungsintervalle usw. über den RFID- und/oder den NFC-Transponder aus einer Datenbank abrufen.

In einer weiteren besonders vorteilhaften Ausführungsform des erfindungsgemäßen Rucksackes oder Koffers sind an einer Außenseite der Oberschale Magnetschnappverschlüsse zur manuell lösbaren Aufnahme von Zubehörteilen angeordnet.

Vorteilhafte Ausführungsformen der vorliegenden Erfindung sind im Folgenden anhand von Figuren näher erläutert, wobei
- Figur 1: eine Ausführungsform eines erfindungsgemäßen Rucksackes in geschlossenem Zustand in einer perspektivischen Vorderansicht zeigt;
- Figur 2: den Rucksack aus Figur 1 in aufgeklapptem Zustand zeigt;
- Figur 3: eine Rückseite des Rucksackes aus Figur 1 zeigt;
- Figur 4: schematisch einen Querschnitt einer Unterschale des Rucksackes aus Figur 1 zeigt;
- Figur 5: schematisch einen Querschnitt einer alternativ ausgebildeten Unterschale des erfindungsgemäßen Rucksackes zeigt;
- Figur 6: schematisch einen Querschnitt einer weiteren alternativen Unterschale des erfindungsgemäßen Rucksackes zeigt;
- Figur 7: eine weitere Ausführungsform eines erfindungsgemäßen Rucksackes in geschlossenem Zustand zeigt; und
- Figur 8: schematisch eine Oberschale und eine Unterschale einer Ausführungsform eines erfindungsgemäßen Rucksackes in aufgeklappter Stellung zeigt.

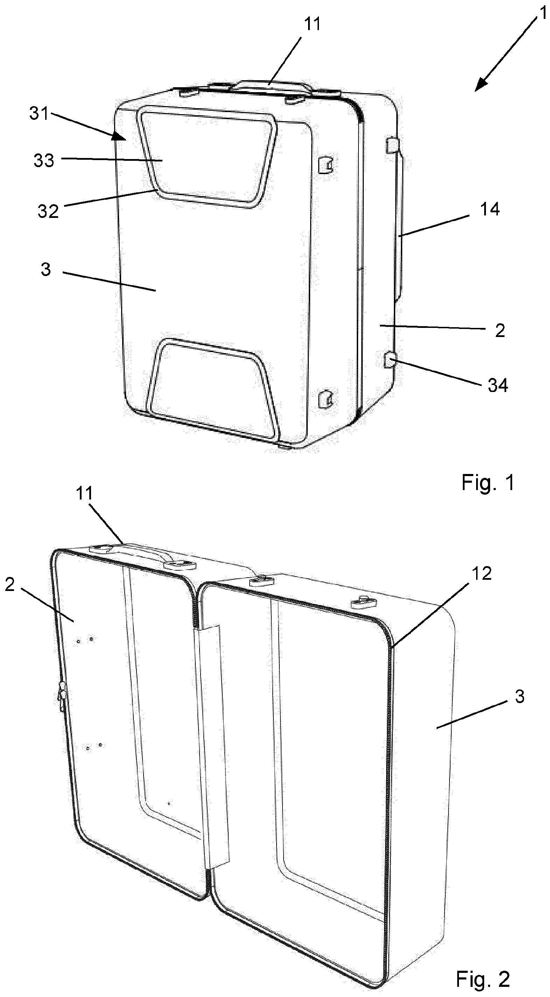

Der in Figur 1 gezeigte Rucksack 1 weist eine Unterschale 2 und eine klappbar mit der Unterschale 2 verbundene Oberschale 3 auf. Die Unterschale 2 als auch die Oberschale 3 sind jeweils als Halbschalen ausgebildet. In Figur 1 ist der Rucksack 1 geschlossen.

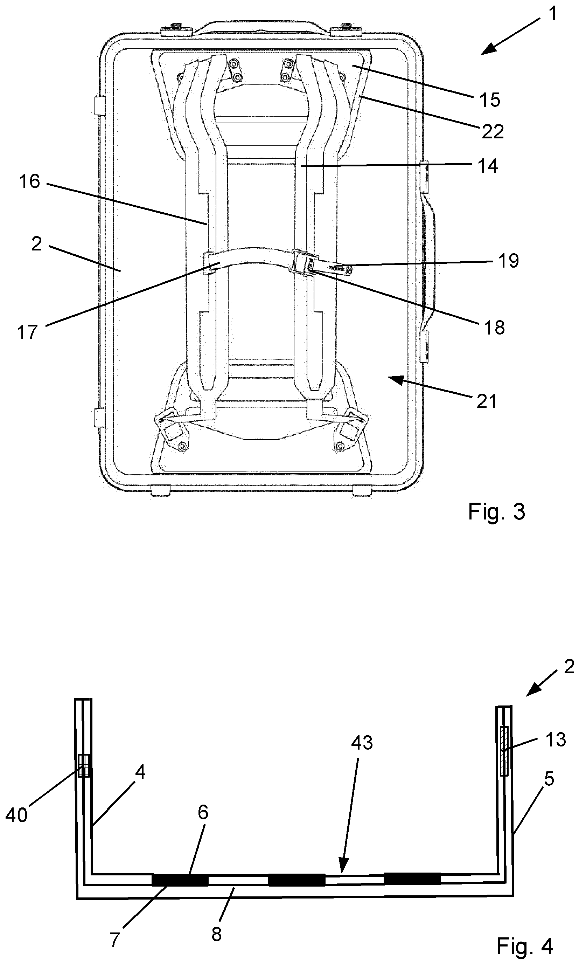

Figur 2 zeigt den Rucksack 1 aus Figur 1 in geöffnetem Zustand. In Figur 3 ist eine Rückseite des Rucksackes 1 aus Figur 1 zu sehen.

Die Oberschale 3 des Rucksackes 1 weist auf ihrer Oberschalenaußenseite 31 in dem gezeigten Ausführungsbeispiel zwei Senken 32 auf, in welchen jeweils eine Reflexionsfolie angeordnet ist, die jeweils von einer transparenten Deckplatte 33, wie einer 1 bis 2 mm dicken Polycarbonatscheibe, abgedeckt ist.

In einer alternativen Ausführungsform des erfindungsgemäßen Rucksackes 1 hat der Rucksack 1 keine entsprechenden Senken 32. Stattdessen ist dann die Reflexionsfolie auf die Oberschalenaußenseite 31 aufgeklebt und nicht von einer transparenten Deckplatte 33 abgedeckt.

Figur 4 zeigt schematisch einen Querschnitt der Unterschale 2 des Rucksackes 1. Die Unterschale 2 weist eine Innenlage 4 und eine Außenlage 5 auf.

Die Innenlage 4 ist aus tiefgezogenem Kunststoff ausgebildet. Die Innenlage 4 kann jedoch auch als Spritzgussteil ausgebildet sein.

Die Innenlage 4 weist die Form einer Halbschale mit darin ausgebildeten, voneinander beabstandeten Vertiefungen 7 auf. Beispielsweise können die Vertiefungen 7 kreisrund ausgebildet sein, sie können jedoch auch streifenförmig ausgebildet sein oder eine andere Form aufweisen, die zur Aufnahme von Elementen geeignet ist. In der gezeigten Ausführungsform sind beispielsweise 35 kreisrunde Vertiefungen 7 mit einem Durchmesser von 20 mm und einer Tiefe von 2 mm ausgebildet.

Die Außenlage 5 ist in der gezeigten Ausführungsform auch aus tiefgezogenem Kunststoff ausgebildet. Sie kann in anderen, nicht gezeigten Ausführungsform der vorliegenden Erfindung auch aus Planenmaterial ausgebildet sein.

Zwischen der Innenlage 4 und der Außenlage 5 befinden sich magnetische Kopplungselemente 6. In der gezeigten Ausführungsform sind die magnetischen Kopplungselemente 6 Magnete. In anderen Ausführungsformen der vorliegenden Erfindung können anstelle der Magnete auch Stahlscheiben als magnetische Kopplungselemente 6 verwendet werden.

Die Vertiefungen 7 sind in einer zu der Außenlage 5 ausgerichteten Außenseite 41 der Innenlage 4 eingebracht. Die Vertiefungen 7 erstrecken sich von der Außenseite 41 in Richtung eines Inneren 10 des Rucksackes 1.

Die magnetischen Kopplungselemente 6 sind in die Vertiefungen 7 der Innenlage 4 eingeklebt. Sie sind dadurch nicht verschiebbar. Die Vertiefungen 7 sind in der gezeigten Ausführungsform an die Form der magnetischen Kopplungselemente 6 angepasst.

Zwischen Innenlage 4 und Außenlage 5 ist großflächig Kleber 8 eingebracht, wodurch die Innenlage 4 mit der Außenlage 5 verklebt ist. In anderen Ausführungsformen des Rucksackes 1 kann die Innenlage 4 auch mittels einer Schweißverbindung mit der Außenlage 5 verbunden sein. Vorzugsweise sind die Innenlage 4 und die Außenlage 5 flüssigkeitsdicht miteinander verbunden.

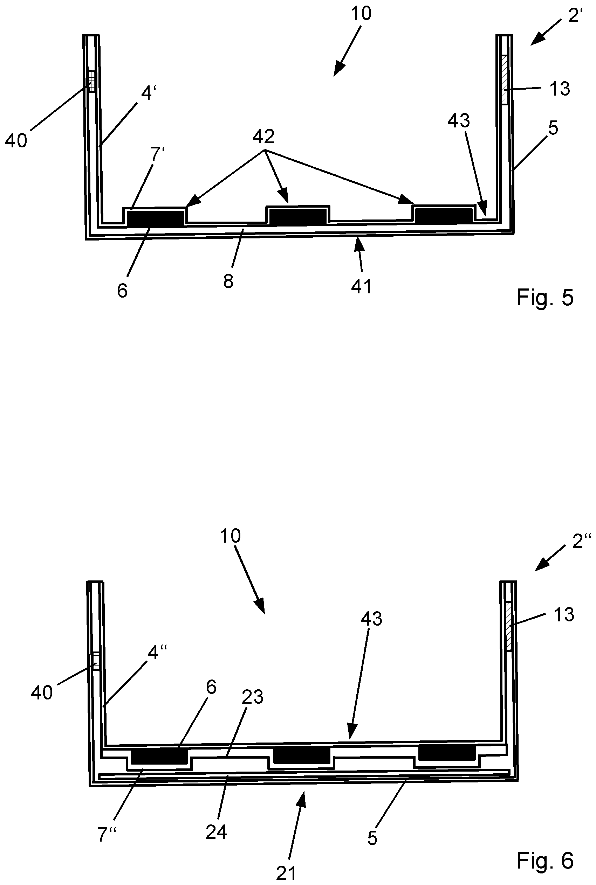

In der in Figur 5 gezeigten Ausbildung der Unterschale 2' des Rucksackes 1 bilden die Vertiefungen 7' noppenartige Auswölbungen 42 an einer durch die Innenlage 4' ausgebildeten Innenseite 43 des Rucksackes 1 aus. In den noppenartigen Auswölbungen 42 sind die magnetischen Kopplungselemente 6 eingebracht. Dadurch dass sich an der Innenseite 43 des Rucksackes 1 Umrisse der magnetischen Kopplungselemente 6 in Form der noppenartigen Auswölbungen 42 abzeichnen, können Ausrüstungsgegenstände zuverlässig an den magnetischen Kopplungselementen 6 positioniert und fixiert werden.

Die Vertiefungen 7' sind in ihrer Form an die aufzunehmenden magnetischen Kopplungselemente 6 angepasst, sodass die magnetischen Kopplungselemente 6 passgenau in die Vertiefungen 7' eingebracht sind. Die Vertiefungen 7' bilden mit den magnetischen Kopplungselementen 6 eine formschlüssige Verbindung aus, sodass die magnetischen Kopplungselemente 6 in den Vertiefungen 7' nicht verrutschen können. Für einen besonders guten Halt der magnetischen Kopplungselemente 6 in den Vertiefungen 7' sind die magnetischen Kopplungselemente 6 in der in Figur 5 gezeigten Ausführungsform des erfindungsgemäßen Rucksackes 1 in die Vertiefungen 7' eingeklebt.

Ferner sind die Vertiefungen 7' in der gezeigten Ausführungsform derart ausgebildet, dass die magnetischen Kopplungselemente 6 bündig mit einer Außenseite der Innenlage 4' in den Vertiefungen 7' aufgenommenen sind und nicht aus den Vertiefungen 7' herausragen. Vorteilhafterweise zeichnen sich die magnetischen Kopplungselemente 6 hierdurch nicht in der angrenzenden Außenlage 5 ab oder wölben die Außenlage 5 nicht nach außen auf.

Die Außenlage 5 ist in der in Figur 5 gezeigten Ausführungsform des erfindungsgemä-βen Rucksackes 1 aus einer thermoplastischen Polyurethan-Plane ausgebildet.

Wie es außerdem in den Figuren 4 und 5 zu sehen ist, ist zwischen der Außenlage 5 und der Innenlage 4 der Unterschale 2 ein RFID-Transponder 40 angeordnet. Anstelle des oder zusätzlich zu dem RFID-Transponder 40 kann hier auch ein NFC-Transponder angeordnet sein. In der gezeigten Ausführungsform des Rucksackes 1 befindet sich der RFID-Transponder 40 an einer Längsseite des Rucksackes 1, genauso gut kann der RFID-Transponder 40 jedoch auch an einer die Längsseiten verbindenden Querseite des Rucksackes 1 angeordnet sein. Ferner kann der Rucksack 1 auch mehr als einen RFID-Transponder 40 aufweisen.

In den gezeigten Ausführungsformen weist die Oberschale 3 einen gleichen Aufbau wie die Unterschale 2 auf. In anderen Ausführungsformen der vorliegenden Erfindung kann die Oberschale 3 auch nur aus einer Schale ausgebildet sein und keine magnetischen Kopplungselemente aufweisen.

In der Ausführungsform der Figuren 1 und 2 sind die Oberschale 3 und die Unterschale 2 mittels eines Reißverschlusses 12 verbunden bzw. verbindbar.

Figur 6 zeigt schematisch einen Querschnitt einer weiteren alternativen Unterschale 2" des erfindungsgemäßen Rucksackes 1. In dieser Ausführungsform ist zwischen der Innenlage 4" und der Außenlage 5 des Rucksackes 1 eine Kunststoffplatte 23 angeordnet. Die Kunststoffplatte 23 weist Vertiefungen 7" auf, in welche die magnetischen Kopplungselemente 6 eingebracht sind. Die magnetischen Kopplungselemente 6 sind bündig in den Vertiefungen 7" aufgenommen und lagefixiert angeordnet.

Die Vertiefungen 7" ragen, anders als in den Ausführungsformen der Figuren 4 und 5, nicht in das Innere 10 des Rucksackes 1. Die Vertiefungen 7" der Figur 6 erstrecken sich in Richtung einer Unterschalenaußenseite 21.

Die magnetischen Kopplungselemente 6 sind passgenau in die Vertiefungen 7" eingebracht, wobei diese auf gleicher Höhe wie die Vertiefungen 7" abschließen. Die magnetischen Kopplungselemente 6 ragen in der in Figur 6 gezeigten Ausführungsform des Rucksackes 1 nicht aus den Vertiefungen 7" heraus. In weiteren Varianten des Rucksackes 1 können die magnetischen Kopplungselemente 6 auch aus den Vertiefungen 7" herausragen und dadurch noppenartige Auswölbungen 42 an der Innenseite 43 des Rucksackes 1 abbilden.

In der in Figur 6 gezeigte Ausführungsform sind die magnetischen Kopplungselemente 6 in die Vertiefungen 7" eingeklebt. In weiteren Ausführungsformen können die magnetischen Kopplungselemente 6 jedoch auch lediglich durch eine zwischen den Vertiefungen 7" und den magnetischen Kopplungselementen 6 ausgebildete formschlüssige Verbindung in den Vertiefungen 7" gehalten werden.

Die Innenlage 4" und die Außenlage 5 der Ausführungsform von Figur 6 sind jeweils aus einer thermoplastischen Polyurethan-Plane ausgebildet. Alternativ können die Innenlage 4" und/oder die Außenlage 5 jedoch auch als Kunststoffformteil oder Kunststoffspritzteil ausgebildet sein.

Zwischen der Außenlage 5 und der Kunststoffplatte 23 ist eine Schaumplatte 24 angeordnet, die in den Rucksack 1 eingebrachte Ausrüstungsgegenstände gut gegen Stöße schützt. Die Schaumplatte 24 ist in ihren Abmessungen den Abmessungen der Innenlage 4" angepasst. In alternativen Ausführungsformen des Rucksackes 1 können auch mehrere nebeneinander und/oder übereinander angeordnete Schaumplatten 24 zwischen der Kunststoffplatte 23 und der Außenlage 5 angeordnet sein. Ebenso sind auch Ausführungsformen des Rucksackes 1 möglich, in denen keine Schaumplatte 24 zwischen der Kunststoffplatte 23 und der Außenlage 5 vorgesehen ist.

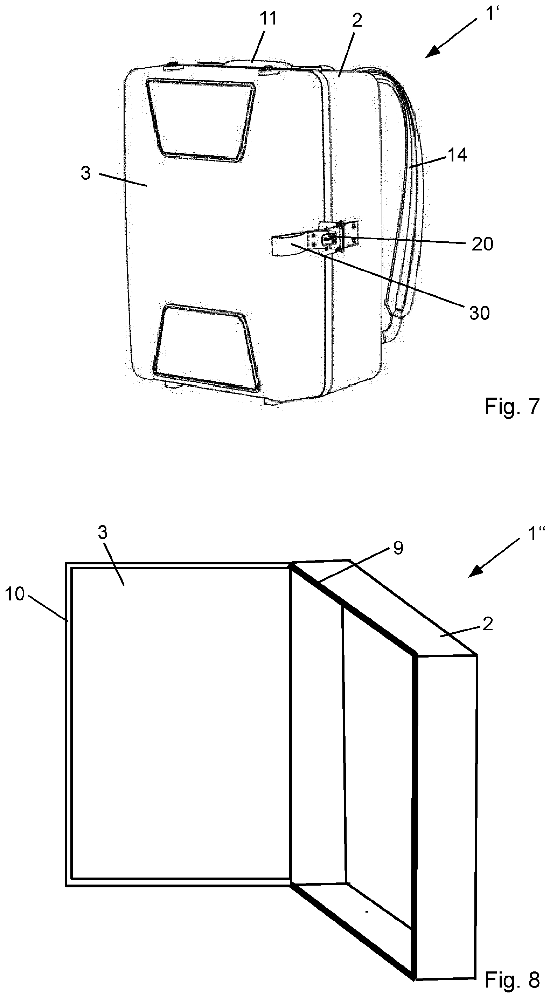

Figur 7 zeigt eine weitere Ausführungsform eines erfindungsgemäßen Rucksackes 1' in geschlossenem Zustand.

Bei dem Rucksack 1' sind an aneinandergrenzenden Seiten der Unterschale 2 und der Oberschale 3 miteinander korrespondierende Teile eines Magnetschnappverschlusses 20 montiert.

Ferner ist bei dem Rucksack 1' an einem Rand der Oberschale 3 eine Zugschlaufe 30 montiert.

Die Rucksäcke 1, 1' weisen jeweils einen Tragegriff 11 auf. Der Tragegriff 11 ist jeweils mit wenigstens einer zwischen der Innenlage 4 und der Außenlage 5 der an der Unterschale 2 angeordneten Verstärkungsplatte 13, welche schematisch in Figur 4 dargestellt ist, verschraubt.

Der Tragegriff 11 ist aus nur einem Materialstreifen ausgebildet.

In anderen Ausführungsformen der vorliegenden Erfindung kann der jeweilige Rucksack auch einen Tragegriff aufweisen, der mittels einer Rastnase in einer zwischen der Innenlage und der Außenlage der Oberschale und/oder der Unterschale angeordneten Tragegriff-Anbindungsplatte angeordnet ist.

Die Rucksäcke 1, 1' weisen jeweils auf ihrer Rückseite einen Schultergurt 14 auf, der aus nur einem Materialstreifen ausgebildet ist. Der jeweilige Schultergurt 14 ist anatomisch vorgeformt.

Wie es in Figur 3 zu sehen ist, ist der Schultergurt 14 an einer Schultergurtbefestigungsplatte 15 befestigt, die in eine auf der Unterschalenaußenseite 21 der Unterschale 2 ausgebildeten Absenkung 22 geklebt ist. In der gezeigten Ausführungsform ist die Schultergurtbefestigungsplatte 15 als ein Zweikomponenten-Spritzguss-Teil mit einer Halteplatte und einer flächig damit verbundenen Dämpfungsplatte ausgebildet ist, wobei die Halteplatte an der Unterschalenaußenseite angeordnet ist.

An dem Schultergurt 14 ist eine einstückig mit dem Schultergurt 14 ausgebildete Kederschiene 16 ausgebildet. Wie es in Figur 3 zu sehen ist, ist an der Kederschiene 16 ein Brustgurt 17 befestigt. An dem Brustgurt 17 ist in der gezeigten Ausführungsform ein Magnetverschluss 18 ausgebildet.

Der Brustgurt 17 ist aus einem dehnbaren Material ausgebildet. Eine Längenverstellung des Brustgurtes 17 wird ausschließlich über die Dehnbarkeit dieses Materials realisiert.

Bei den Rucksäcken 1, 1' sind jeweils die Dämpfungsplatte der Schultergurtbefestigungsplatte 15, der Schultergurt 14, der Tragegriff 11, der Brustgurt 17 und Zugfähnchen 19, soweit vorhanden, jeweils in einem Stück aus einem geschlossenporigen, flexiblen und dehnbaren Material ausgebildet.

Ferner können an einer Außenseite der Oberschale 3 Magnetschnappverschlüsse zur manuell lösbaren Aufnahme von Zubehörteilen angeordnet sein.

Zudem sind an der Oberschale 3 und der Unterschale 2 der Rucksäcke 1, 1' Füßchen 34 ausgebildet oder montiert.

Figur 8 zeigt schematisch eine Oberschale 3 und eine Unterschale 2 einer Ausführungsform eines erfindungsgemäßen Rucksackes 1", der ähnlich wie der Rucksack 1' von Figur 7 ausgebildet ist, in aufgeklapptem Zustand. Bei dieser Ausführungsform bildet ein Rand 9 der Unterschale 2 eine Feder, die zum Eingreifen in einen als Nut ausgebildeten Rand 10 der Oberschale 3 ausgebildet ist.

## Patentansprüche

1. Rucksack oder Koffer (1, 1', 1") mit einer Unterschale (2, 2', 2") und einer klappbar mit der Unterschale (2, 2', 2") verbundenen Oberschale (3), wobei sowohl die Unterschale (2, 2', 2") als auch die Oberschale (3) jeweils eine Innenlage (4, 4', 4") und eine Außenlage (5) aufweisen und die Oberschale (3) und/oder die Unterschale (2, 2', 2") magnetische Kopplungselemente (6) aufweist/aufweisen, **dadurch gekennzeichnet, dass** die Innenlage (4, 4', 4") der Oberschale (3) und/oder der Unterschale (2, 2', 2") eine Kunststoffwanne mit Vertiefungen (7, 7', 7") aufweist, in welchen die magnetischen Kopplungselemente (6) bündig aufgenommen und lagefixiert angeordnet sind, wobei sich die magnetischen Kopplungselemente (6) zwischen der Außenlage (5) und der Innenlage (4, 4', 4") der Oberschale (3) und/oder zwischen der Außenlage (5) und der Innenlage (4, 4', 4") der Unterschale (2) befinden oder zwischen der Innenlage (4") und der Außenlage (5) eine Kunststoffplatte (23) mit Vertiefungen (7") angeordnet ist, in welchen die magnetischen Kopplungselemente (6) bündig aufgenommen und lagefixiert angeordnet sind.

2. Rucksack oder Koffer nach Anspruch 1, **dadurch gekennzeichnet, dass** die magnetischen Kopplungselemente (6) in die Vertiefungen (7, 7', 7") geklebt sind.

3. Rucksack oder Koffer nach einem der vorhergehenden Ansprüche, **dadurch gekennzeichnet, dass** die Innenlage (4, 4', 4") und die Außenlage (5) der Oberschale (3) und der Unterschale (2, 2', 2") flüssigkeitsdicht miteinander verklebt oder verschweißt sind.

4. Rucksack oder Koffer nach einem der vorhergehenden Ansprüche, **dadurch gekennzeichnet, dass** ein Rand (9) der Unterschale (2, 2', 2") und ein Rand (10) der Oberschale (3) zusammen eine Nut-Feder-Verbindung ausbilden.

5. Rucksack oder Koffer nach einem der vorhergehenden Ansprüche, **dadurch gekennzeichnet, dass** der Rucksack oder Koffer (1, 1', 1") wenigstens einen Tragegriff (11) aufweist, der mit wenigstens einer zwischen der Innenlage (4, 4', 4") und der Außenlage (5) der Oberschale (3) und/oder der Unterschale (2, 2', 2") angeordneten Verstärkungsplatte (13) verschraubt ist.

6. Rucksack oder Koffer nach einem der vorhergehenden Ansprüche, **dadurch gekennzeichnet, dass** der Rucksack oder Koffer (1, 1', 1") wenigstens einen Tragegriff (11) aufweist, der aus nur einem Materialstreifen ausgebildet ist und mittels einer Rastnase in einer zwischen der Innenlage (4, 4', 4") und der Außenlage (5) der Oberschale (3) und/oder der Unterschale (2, 2', 2") angeordneten Tragegriff-Anbindungsplatte angeordnet ist.

7. Rucksack oder Koffer nach einem der vorhergehenden Ansprüche, **dadurch gekennzeichnet, dass** der Rucksack oder Koffer (1, 1', 1") wenigstens einen Schultergurt (14) aufweist, der aus nur einem Materialstreifen ausgebildet ist.

8. Rucksack oder Koffer nach Anspruch 7, **dadurch gekennzeichnet, dass** der wenigstens eine Schultergurt (14) an einer Schultergurtbefestigungsplatte (15) befestigt ist, die zwischen der Innenlage (4, 4', 4") und der Außenlage (5) der Unterschale (2) angeordnet ist oder die in eine auf einer Unterschalenaußenseite (21) der Unterschale (2) ausgebildeten Absenkung (22) geklebt ist.

9. Rucksack oder Koffer nach einem der Ansprüche 7 oder 8, **dadurch gekennzeichnet, dass** an dem wenigstens einen Schultergurt (14) eine einstückig mit dem Schultergurt (14) ausgebildete Kederschiene (16) ausgebildet ist.

10. Rucksack oder Koffer nach Anspruch 9, **dadurch gekennzeichnet, dass** ein Brustgurt (17) an der Kederschiene (16) befestigt ist, an dem ein Magnetverschluss (18) ausgebildet ist, wobei der Brustgurt (17) aus einem dehnbaren Material ausgebildet ist und eine Längenverstellung des Brustgurtes (17) ausschließlich über die Dehnbarkeit dieses Materials realisiert wird.

11. Rucksack oder Koffer nach einem der Ansprüche 7 bis 10, **dadurch gekennzeichnet, dass** der Schultergurt (14), der wenigstens eine Tragegriff (11), der Brustgurt (17) und Zugfähnchen (19), soweit vorhanden, jeweils in einem Stück aus einem geschlossenporigen, flexiblen und dehnbaren Material ausgebildet sind.

12. Rucksack oder Koffer nach einem der vorhergehenden Ansprüche, **dadurch gekennzeichnet, dass** an aneinandergrenzenden Seiten der Unterschale (2, 2', 2") und der Oberschale (3) miteinander korrespondierende Teile eines Magnetschnappverschlusses (20) montiert sind.

13. Rucksack oder Koffer nach einem der vorhergehenden Ansprüche, **dadurch gekennzeichnet, dass** zwischen der Außenlage (5) und der Kunststoffplatte (23) wenigstens eine Schaumplatte (24) angeordnet ist.

14. Rucksack oder Koffer nach einem der vorhergehenden Ansprüche, **dadurch gekennzeichnet, dass** zwischen der Außenlage (5) und der Innenlage (4, 4') der Oberschale (3) und/oder der Unterschale (2, 2') ein RFID- und/oder ein NFC-Transponder (40) angeordnet ist/sind.

15. Rucksack oder Koffer nach einem der vorhergehenden Ansprüche, **dadurch gekennzeichnet, dass** an einer Außenseite der Oberschale (3) Magnetschnappverschlüsse zur manuell lösbaren Aufnahme von Zubehörteilen angeordnet sind.

## Claims

1. Rucksack or suitcase (1, 1', 1") with a lower shell (2, 2', 2") and an upper shell (3) which is foldably connected to the lower shell (2, 2', 2"), wherein both the lower shell (2, 2', 2") and the upper shell (3) comprise an inner layer (4, 4', 4") and an outer layer (5) and the upper shell (3) and/or the lower shell (2, 2', 2") comprise(s) magnetic coupling elements (6), **characterized in that** the inner layer (4, 4', 4") of the upper shell (3) and/or of the lower shell (2, 2', 2") comprise(s) a plastic tray with recesses (7, 7', 7") in which the magnetic coupling elements (6) are received flush and arranged in a fixed position, wherein the magnetic coupling elements (6) are located between the outer layer (5) and the inner layer (4, 4', 4") of the upper shell (3) and/or between the outer layer (5) and the inner layer (4, 4', 4") of the lower shell (2), or between the inner layer (4") and the outer layer (5) a plastic plate (23) with recesses (7") is arranged, in which the magnetic coupling elements (6) are received flush and arranged in a fixed position.

2. Rucksack or suitcase according to claim 1, **characterized in that** the magnetic coupling elements (6) are glued into the recesses (7, 7', 7").

3. Rucksack or suitcase according to one of the preceding claims, **characterized in that** the inner layer (4, 4', 4") and the outer layer (5) of the upper shell (3) and the lower shell (2, 2', 2") are glued or welded together in a liquid-tight manner.

4. Rucksack or suitcase according to one of the preceding claims, **characterized in that** an edge (9) of the lower shell (2, 2', 2") and an edge (10) of the upper shell (3) together form a tongue and groove connection.

5. Rucksack or suitcase according to one of the preceding claims, **characterized in that** the rucksack or suitcase (1, 1', 1") comprises at least one carrying handle (11) which is screwed to at least one reinforcing plate (13) arranged between the inner layer (4, 4', 4") and the outer layer (5) of the upper shell (3) and/or the lower shell (2, 2', 2").

6. Rucksack or suitcase according to one of the preceding claims, **characterized in that** the rucksack or suitcase (1, 1', 1") comprises at least one carrying handle (11) which is formed from only one strip of material and is arranged by means of a locking lug in a carrying handle connection plate arranged between the inner layer (4, 4', 4") and the outer layer (5) of the upper shell (3) and/or the lower shell (2, 2', 2").

7. Rucksack or suitcase according to one of the preceding claims, **characterized in that** the rucksack or suitcase (1, 1', 1") has at least one shoulder strap (14) which is formed from only one strip of material.

8. Rucksack or suitcase according to claim 7, **characterized in that** the at least one shoulder strap (14) is fastened to a shoulder strap fastening plate (15) which is arranged between the inner layer (4, 4', 4") and the outer layer (5) of the lower shell (2) or which is glued into a depression (22) formed on an outer side (21) of the lower shell (2).

9. Rucksack or suitcase according to one of claims 7 or 8, **characterized in that** a keder rail (16) formed in one piece with the shoulder strap (14) is formed on the at least one shoulder strap (14).

10. Rucksack or suitcase according to claim 9, **characterized in that** a chest strap (17) is attached to the keder rail (16), on which a magnetic closure (18) is formed, wherein the chest strap (17) is formed from a stretchable material and a length adjustment of the chest strap (17) is realized exclusively via the stretchability of this material.

11. Rucksack or suitcase according to one of claims 7 to 10, **characterized in that** the shoulder strap (14), the at least one carrying handle (11), the chest strap (17) and pull tabs (19), if present, are each formed in one piece from a closed-pore, flexible and stretchable material.

12. Rucksack or suitcase according to one of the preceding claims, **characterized in that** corresponding parts of a magnetic snap fastener (20) are mounted on adjacent sides of the lower shell (2, 2', 2") and the upper shell (3).

13. Rucksack or suitcase according to one of the preceding claims, **characterized in that** at least one foam plate (24) is arranged between the outer layer (5) and the plastic plate (23).

14. Rucksack or suitcase according to one of the preceding claims, **characterized in that** an RFID and/or an NFC transponder (40) is/are arranged between the outer layer (5) and the inner layer (4, 4') of the upper shell (3) and/or the lower shell (2, 2').

15. Rucksack or suitcase according to one of the preceding claims, **characterized in that** magnetic snap fasteners for manually releasable accommodation of accessories are arranged on an outer side of the upper shell (3).

## Revendications

1. Sac à dos ou valise (1, 1', 1") avec une coque inférieure (2, 2', 2") et une coque supérieure (3) reliée de manière pliable à la coque inférieure (2, 2', 2"), la coque inférieure (2, 2', 2") et la coque supérieure (3) comprenant chacune une couche intérieure (4, 4', 4") et une couche extérieure (5) et la coque supérieure (3) et/ou la coque inférieure (2, 2', 2") comprenant des éléments de couplage magnétiques (6), **caractérisé en ce que** la couche intérieure (4, 4', 4") de la coque supérieure (3) et/ou de la coque inférieure (2, 2', 2") comporte(nt) un plateau en matière plastique comportant des évidements (7, 7', 7") dans lesquels les éléments de couplage magnétiques (6) sont reçus à même niveau et disposés dans une position fixe, les éléments de couplage magnétiques (6) étant situés entre la couche extérieure (5) et la couche intérieure (4, 4', 4") de la coque supérieure (3) et/ou entre la couche extérieure (5) et la couche intérieure (4, 4', 4") de la coque inférieure (2), ou entre la couche intérieure (4") et la couche extérieure (5) une plaque en matière plastique (23) avec des évidements (7") est disposée, dans laquelle les éléments de couplage magnétique (6) sont reçus à même niveau et disposés dans une position fixe.

2. Sac à dos ou valise selon la revendication 1, **caractérisé en ce que** les éléments de couplage magnétique (6) sont collés dans les évidements (7, 7', 7").

3. Sac à dos ou valise selon l'une des revendications précédentes, **caractérisé en ce que** la couche intérieure (4, 4', 4") et la couche extérieure (5) de la coque supérieure (3) et de la coque inférieure (2, 2', 2") sont collées ou soudées ensemble de manière étanche aux liquides.

4. Sac à dos ou valise selon l'une des revendications précédentes, **caractérisé en ce que** un bord (9) de la coque inférieure (2, 2', 2") et un bord (10) de la coque supérieure (3) forment ensemble une liaison à rainure et languette.

5. Sac à dos ou valise selon l'une des revendications précédentes, **caractérisé en ce que** le sac à dos ou la valise (1, 1', 1") comporte au moins une poignée de transport (11) qui est vissée à au moins une plaque de renfort (13) disposée entre la couche intérieure (4, 4', 4") et la couche extérieure (5) de la coque supérieure (3) et/ou de la coque inférieure (2, 2', 2").

6. Sac à dos ou valise selon l'une des revendications précédentes, **caractérisé en ce que** le sac à dos ou la valise (1, 1', 1") comporte au moins une poignée de transport (11) qui est formée d'une seule bande de matériau et qui est disposée au moyen d'un ergot d'enclenche dans une plaque de liaison de poignée de transport disposée entre la couche intérieure (4, 4', 4") et la couche extérieure (5) de la coque supérieure (3) et/ou de la coque inférieure (2, 2', 2").

7. Sac à dos ou valise selon l'une des revendications précédentes, **caractérisé en ce que** le sac à dos ou la valise (1, 1', 1") comporte au moins une bandoulière (14) qui est formée d'une seule bande de matériau.

8. Sac à dos ou valise selon la revendication 7, **caractérisé en ce que** l'au moins une bandoulière (14) est fixée à une plaque de fixation de bandoulières (15) qui est disposée entre la couche intérieure (4, 4', 4") et la couche extérieure (5) de la coque inférieure (2) ou qui est collée dans un creux (22) formé sur un côté extérieur (21) de la coque inférieure (2).

9. Sac à dos ou valise selon l'une des revendications 7 ou 8, **caractérisé en ce que** un rail à bourrelet (16) formé d'une seule pièce avec la bandoulière (14) est formé sur l'au moins une bandoulière (14).

10. Sac à dos ou valise selon la revendication 9, **caractérisé en ce que** une sangle de torse (17) est fixée au rail à bourrelet (16), sur laquelle une fermeture magnétique (18) est formée, la sangle de torse (17) étant formée d'un matériau extensible et un réglage de la longueur de la sangle de torse (17) étant réalisé exclusivement par l'extensibilité de ce matériau.

11. Sac à dos ou valise selon l'une des revendications 7 à 10, **caractérisé en ce que** la bandoulière (14), l'au moins une poignée de transport (11), la sangle de torse (17) et les tirettes (19), si elles sont présentes, sont chacune formées d'une seule pièce à partir d'un matériau à pores fermés, flexible et extensible.

12. Sac à dos ou valise selon l'une des revendications précédentes, **caractérisé en ce que** des parties correspondantes d'une fermeture à déclic magnétique (20) sont montées sur des côtés adjacents de la coque inférieure (2, 2', 2") et de la coque supérieure (3).

13. Sac à dos ou valise selon l'une des revendications précédentes, **caractérisé en ce que** au moins une plaque de mousse (24) est disposée entre la couche extérieure (5) et la plaque en matière plastique (23).

14. Sac à dos ou valise selon l'une des revendications précédentes, **caractérisé en ce que** un transpondeur RFID et/ou NFC (40) est/sont disposé(s) entre la couche extérieure (5) et la couche intérieure (4, 4') de la coque supérieure (3) et/ou de la coque inférieure (2, 2').

15. Sac à dos ou valise selon l'une des revendications précédentes, **caractérisé en ce que** des fermetures à déclic magnétiques pour le logement amovible manuellement d'accessoires sont disposés sur un côté extérieur de la coque supérieure (3).
